# EUROPEAN PATENT SPECIFICATION

(11) **EP 4 212 204 B1**
(45) Date of publication and mention of the grant of the patent: **04.11.2026**
(21) Application number: 22209289.2
(22) Date of filing: 24.11.2022
(51) Int. Cl.: A61N 1/05, A61B 5/00, A61B 5/25, A61N 1/08, A61B 5/055, A61B 5/0507

(54) **MEDICAL ELECTRODE DEVICE FOR IMPLANTATION INTO A PATIENT AND METHOD FOR FABRICATING A MEDICAL ELECTRODE DEVICE**
MEDIZINISCHE ELEKTRODENVORRICHTUNG ZUR IMPLANTATION IN EINEN PATIENTEN UND VERFAHREN ZUR HERSTELLUNG EINER MEDIZINISCHEN ELEKTRODENVORRICHTUNG
DISPOSITIF D'ÉLECTRODE MÉDICALE DESTINÉ À ÊTRE IMPLANTÉ DANS UN PATIENT ET PROCÉDÉ DE FABRICATION D'UN DISPOSITIF D'ÉLECTRODE MÉDICALE

(30) Priority: 18.01.2022 DE 202022100244 U
(43) Date of publication of application: 19.07.2023
(73) Proprietor: BIOTRONIK SE & Co. KG, 12359 Berlin (DE)
(72) Inventor: Rump, Jens, 12049 Berlin (DE)
(74) Representative: Biotronik Corporate Services SE

(56) References cited:
- US-A1- 2011 040 343
- US-A1- 2014 107 746
- US-A1- 2015 039 064
- US-A1- 2015 080 694
- US-A1- 2020 254 235
- US-B1- 7 962 224
- US-B2- 10 155 106
- US-B2- 8 219 199
- US-B2- 8 805 541
- US-B2- 9 492 651

## Description

The present invention relates to a medical electrode device for implantation into a patient and a method for fabricating a medical electrode device.

A medical electrode device of this kind may for example serve for a neuro-stimulation and for this may be implanted into a patient for example in the region of the spinal cord, for example into the epidural space near the spinal cord of the spinal column of a patient. In this way a nerve stimulation at the spinal cord may be achieved by injecting electrical stimulation currents.

An electrode device of the kind concerned herein however may also be used for emitting stimulation signals or receiving sense signals at other locations within a patient, for example within the brain or in cardiac applications.

An electrode device of the kind concerned herein comprises a lead body extending along a longitudinal axis and having a proximal end and a distal end. One or multiple electrical lines extend within the lead body. A flattened electrode end is arranged at the distal end of the lead body and comprises a carrier element and an arrangement of contact elements arranged on a flat face of the carrier element for contacting tissue. The electrical contact elements are electrically connected to the one or the multiple electrical lines extending within the lead body. In an implanted state, the lead body with its proximal end is connected to a generator for generating stimulation signals. The flattened electrode end is implanted in a patient, for example in the epidural space of the spinal column, such that the contact elements of the electrode end are in contact with surrounding tissue and may be used to inject stimulation signals into the tissue in order to provide for a stimulation action in the vicinity e.g. of the spinal cord.

The medical electrode device for example has the shape of a so-called paddle electrode, the flattened electrode end having a paddle-like shape carrying e.g. an arrangement of multiple evenly or unevenly distributed contact elements on its surface for emission of electrical signals into and/or reception of electrical signals from surrounding tissue.

Different designs of paddle electrodes are known, for example, from US 6,895,283, US 2008/0046050 A1, US 2014/0172057 A1 and US 9,561,363.

Generally, a medical electrode device of the kind concerned herein shall be compatible with MRI devices. A patient in which an implant system comprising a medical electrode device is implanted may have to undergo an MRI examination, such that it must be made sure that the patient is not posed with a hazardous risk due to the interaction of the implanted system with the MRI device.

Generally, electrical fields produced by an MRI device may couple into the electrode device, wherein the electrode device with the at least one electrical line extending within the lead body and the flattened electrode end arranged at the distal end of the lead body may exhibit a resonant behavior, causing a coupling of the RF excitation field of the MRI device into the electrode device and an increase of the electrical field strength in the vicinity of the electrode device. This makes it necessary to design the electrode device such that in particular in the region of the flattened electrode end, at which the electrical contact elements shall come into electric contact with surrounding tissue, an excessive heating is prevented.

DE 102020 100 121 A1 discloses an implantable electrode comprising an outer tube having a distal end and a proximal end. At the proximal end the electrode is connectable to an active device. Within the outer tube at least one electrical line is arranged. In the region of the distal end in addition an electrical electrode pole is formed for electrically contacting with tissue in an implanted state of the electrode. In the region of the distal end, herein, an electrical tap line is formed in connection with the electrical line of the electrode such that the electrical length of the electrical line is modified.

US 2014/0135614 A1 discloses an implantable electrical stimulation lead including a lead body having a distal end, a proximal end, and a longitudinal length. A plurality of electrodes is disposed along the distal end of the lead body. A plurality of terminals is disposed along the proximal end of the lead body. A plurality of conductors electrically couples the plurality of electrodes to the plurality of terminals. To reduce or redistribute current induced in the conductors during an MRI procedure, an internal conductive structure, such as a dummy coil or hollow metal tube, may be provided. US 2014/107746; US 2011/040343; US 2015/039064 and US 2020/254235 also disclose devices according to the prior art.

It is an object of the instant invention to provide a medical electrode device and a method for fabricating a medical electrode device which allow for a reduced risk of an excessive heating in particular in the vicinity of the flattened electrode end due to RF excitation fields during an MRI examination, while allowing for an easy and cost-efficient production of the electrode device.

This object is achieved by means of a medical electrode device comprising the features of claim 1.

Accordingly, a medical electrode device for implantation into a patient comprises a lead body extending longitudinally along a longitudinal axis. At least one electrical line extends within the lead body. A flattened electrode end is arranged at a distal end of the lead body and comprises a carrier element and a multiplicity of electrical contact elements connected to the at least one electrical line, wherein the multiplicity of electrical contact elements is arranged on a flat face of the carrier element for contacting tissue in proximity to the flattened electrode end in an implanted state of the medical electrode device. The flattened electrode end further comprises an antenna element arranged on the carrier element and comprising an antenna line having a first end and a second end, wherein the antenna line extends along a flat plane.

The medical electrode device for example may form a so-called paddle electrode which may be used for example for a neuro-stimulation device. The medical electrode device in particular may be designed for connection to an active device at its proximal end, the active device serving as a stimulation device for generating electrical stimulation signals which, via the at least one electrical line, are provided to the arrangement of electrical contact elements on the flattened electrode end for causing an electrical stimulation in the region of the flattened electrode end.

The flattened electrode end, herein, in an implanted state rests within tissue of a patient, for example in the region of the spinal cord, for example in the epidural space near the spinal cord of the spinal column of the patient, in order to allow a nerve stimulation at the spinal cord by injecting electrical stimulation currents via the arrangement of electrical contact elements on the flattened electrode end.

The at least one electrical line extending within the lead body together with the electrical contact elements arranged on the flattened electrode end forms an electrical structure, which may be resonant at RF frequencies used within an MRI examination. For example, during an MRI examination using an MRI device at a magnetic field strength of 1.5 Tesla, an RF excitation field is produced at the so-called lamor frequency of 63,87 MHz. At an MRI magnetic field strength of 3 Tesla, the RF excitation field is at a frequency of 127.74 MHz. If the electrical structure formed by the at least one electrical line extending along the lead body and the electrical contact elements arranged on the flattened electrode end is resonant at the RF excitation frequency, the excitation field may couple into the electrical structure and may cause a substantial field increase in the region of the electrode device. Because the electrical contact elements are designed to contact, in an implanted state, with tissue in the vicinity of the flattened electrode end, the field increase on the electrode device may cause a heating of tissue, which however shall be prevented.

For this reason, the flattened electrode end comprises an antenna element arranged on the carrier element in addition to the electrical contact elements. The antenna element herein is formed by an antenna line extending along a flat plane. The antenna element in particular may be designed such that electrical fields predominantly couple into the antenna element, wherein the antenna element may be arranged within the carrier element such that it does not come into electric contact with surrounding tissue, thus allowing to reduce an impact on the surrounding tissue. Furthermore, the antenna element extending along the flat plane on the carrier element may extend over a significant portion of the carrier element of the flattened electrode end, such that heat dissipation may be distributed spatially across the carrier element, hence preventing a pronounced local heating effect on the carrier element.

Because the antenna element may be confined to the flattened electrode end, the fabrication of the electrode device becomes easy. The antenna element may be provided as a flat structure and may be embedded within the carrier element. The flattened electrode end herein may be fabricated separately from the lead body, wherein the antenna element is included in the flattened electrode end, without having to modify the fabrication of the lead body.

The antenna element serves as a passive element which, by design, prevents an excessive heating at the flattened electrode end. In particular, the antenna element may be designed such that electrical fields of an RF excitation field at a predefined MRI magnetic field strength, e.g. 1.5 Tesla or 3 Tesla, predominantly couple into the antenna element, but less into the electrical structure formed by the at least one electrical line extending along the lead body and the electrical contact elements arranged on the flattened electrode end. The antenna element rests within the carrier element and does not come into contact with tissue and in addition provides for an energy dissipation over a fairly large region in the surrounding of the flattened electrode end, hence preventing an excessive heating at the flattened electrode end.

In one embodiment, the flat plane along which the antenna line extends is oriented substantially in parallel to the flat face of the carrier element. The electrical contact elements are arranged on the flat face of the carrier element in order to come into contact with tissue in an implanted state of the medical electrode device. The antenna line of the antenna element extends in parallel to said flat face, but beneficially not on the surface of the carrier element. Rather, beneficially the antenna line extends within the carrier element, the antenna line hence being embedded within the carrier element.

In one embodiment, the antenna line extends along a meandering path, a zig-zag path, a spiral path, a path of a Hilbert curve, or a path of a Peano curve along said flat plane. The antenna line hence does not (fully) extend linearly, but comprises a multiplicity of curves, turns or kinks. In this way, the antenna element is formed in a planar fashion along the flat plane, but may comprise a substantial physical (and electrical) length in that the antenna line forms a meandering path, a zig-zag path, a spiral path, a path of a Hilbert curve or a path of a Peano curve along the flat plane.

In one embodiment, the antenna element comprises a first layer and a second layer formed by the antenna line. The first layer and the second layer herein at least partially extend in parallel to one another. The first layer hence may extend along a first flat plane, whereas the second layer extends along a second flat plane in parallel to said first flat plane. The first layer and the second layer, when viewed along a projection direction perpendicular to the flat plane, may fully or partially overlap.

In one embodiment, the first layer and the second layer are folded with respect to one another at a fold line, the first layer and the second layer extending in parallel to one another from the fold line.

When the antenna element comprises different layers folded with respect to one another and extending in parallel to one another, the overall antenna element still extends along a common flat plane. The layers of the antenna element each extend flatly and are placed on top of one another, wherein an electrical insulation in between the different layers of the antenna element (formed by the same antenna line or by connected sections of multiple lines) may be provided for example by placing an electrically insulating layer in between the layers, for example an electrically insulating foil, for example a plastics foil. Alternatively, the antenna line forming the antenna element may for example comprise an electrically insulating coating, such as a polyurethane (PU) coating or a parylene coating, such that the antenna line is electrically insulated along its length.

In one embodiment, the antenna line is formed by an electrical wire or cable.

In one embodiment, the antenna line is formed by a flexible conducting path, in particular made from a metallic foil. The metallic foil may be cut by a laser to form a line pattern such that the antenna line for example follows a meandering, zig-zag or in another way curved path.

In one embodiment, the antenna line comprises a physical length equal to or larger than 150 mm, in particular larger than 200 mm, for example larger than 300 mm. In that the antenna line may extend along a meandering path, a zig-zag path or another curved path, the length of the antenna line is measured along its path. The physical length of the antenna line in particular may be (much) larger than the physical outer dimensions of the antenna element as measured along the flat plane.

In one embodiment, the antenna line is designed such that, in a state corresponding to the implanted state of the medical electrode device, it exhibits a resonant behavior, in particular a series resonance, at the lamor frequency of a predefined MRI magnetic field strength, in particular 1.5 Tesla or 3 Tesla. In particular, the antenna line may be designed such that it has an electrical length equal to a half wavelength or an integer multiple of a half wavelength at the lamor frequency at a predefined MRI magnetic field strength, in particular 1.5 Tesla or 3 Tesla. The electrical length herein is determined by assuming conditions that arise in the implanted state of the electrode device. In particular, it can be assumed that, for implantation in the region of the spinal cord, the electrode device primarily rests within connective tissue and fat tissue having a relative permittivity of about 60 and an electrical conductivity of about 0.47 S/m at 63.87 MHz, corresponding to the lamor frequency at a magnetic field strength of 1.5 Tesla. By assuming such values for computing the electrical length, the antenna line may be designed such that it exhibits a resonant behavior at the frequency in question.

As electrical fields within a patient's body generally experience damping, it may not be necessary to design the electrical length of the antenna line to exactly correspond to a half wavelength or an integer multiple of a half wavelength at a predefined frequency. Rather, due to the electrical damping a resonant peak may broaden, making it sufficient to assume approximate values e.g. for the relative permittivity and the electrical conductivity. In addition, due to the damping even at an electrical length corresponding to for example a quarter wavelength a sufficient coupling to the antenna element by the RF excitation field during an MRI examination may be achieved.

In one embodiment, the first end and the second end of the antenna line both are arranged within the carrier element such that the antenna element is fully confined to the flattened electrode end. The antenna element hence is formed on the carrier element of the flattened electrode end. This makes it possible to fabricate the flattened electrode end and the lead body of the electrode device separately from one another, wherein the fabrication of the lead body is not influenced by the fabrication of the flattened electrode end, hence easing the fabrication of the overall structure of the electrode device.

In one embodiment, the antenna element is electrically open at the first end and the second end. Beneficially, herein, the antenna element is electrically connected neither to the electrical contact elements nor to the at least one electrical line nor to any other conductive structure of the electrode device. The antenna element hence is electrically separated from other electrical elements of the electrode device, in particular the electrical contact elements of the flattened electrode end and the at least one electrical line extending along the lead body. The antenna element, hence, as a passive element serves as a receiving structure for predominantly coupling to an RF excitation field as produced during an MRI examination.

In one embodiment, the carrier element comprises a body, the antenna element being embedded within the body such that the antenna element is fully received within the body and is electrically insulated towards the outside of the body. The body for example may be made from an electrically insulating material, wherein the antenna element is fully embedded within the material of the body such that it does not come into electric contact with surrounding tissue in an implanted state of the electrode device. In one embodiment, the antenna element is arranged on a portion of the body at a face opposite to the flat face on which the electric contact elements are arranged. The antenna element on the portion of the body is covered by an electrically insulating layer connected to the portion of the body (e.g. by gluing or welding), such that the antenna element is embedded and enclosed within the body.

The body and/or the portion of the body and/or the layer covering the portion of the body may for example be formed from a silicone material or a polyurethane (PU) material.

In another aspect, a method for fabricating a medical electrode device for implantation into a patient comprises: providing a lead body extending longitudinally along a longitudinal axis, at least one electrical line extending within the lead body; and arranging a flattened electrode end at a distal end of the lead body, the flattened electrode end comprising a carrier element and a multiplicity of electrical contact elements connected to the at least one electrical line, wherein the multiplicity of electrical contact elements is arranged on a flat face of the carrier element for contacting tissue in proximity to the flattened electrode end in an implanted state of the medical electrode device, wherein the flattened electrode end further comprises an antenna element arranged on the carrier element and comprising an antenna line having a first end and a second end, wherein the antenna line extends along a flat plane.

The advantages and advantageous embodiments described above for the medical electrode device equally apply also to the method.

The various features and advantages of the present invention may be more readily understood with reference to the following detailed description and the embodiments shown in the drawings. Herein,
- Fig. 1: shows a view of an electrode device connected to a stimulation device in an implanted state in the area of the spine of a patient;
- Fig. 2: shows a view of the electrode device in the epidural space in the region of the spinal column;
- Fig. 3: shows a view of a flattened electrode end of an embodiment of an electrode device;
- Fig. 4: shows another view of a flattened electrode end of a medical electrode device;
- Fig. 5: shows a schematic drawing of an electrode device in an implanted state within a patient;
- Fig. 6: shows an embodiment of a flattened electrode end of an electrode device having an arrangement of electrical contact elements and an antenna element;
- Fig. 7: shows a schematic view of a flat face of the flattened electrode end with electrical contact elements arranged thereon;
- Fig. 8: shows an embodiment of an antenna element embedded within a carrier element of the flattened electrode end;
- Fig. 9: shows another embodiment of an antenna element embedded within a carrier element of the flattened electrode end;
- Fig. 10: shows yet another embodiment of an antenna element;
- Fig. 11: shows yet another embodiment of an antenna element;
- Fig. 12: shows yet another embodiment of an antenna element;
- Fig. 13: shows yet another embodiment of an antenna element; and
- Fig. 14: shows an embodiment of an antenna element having folded layers.

Subsequently, embodiments of the invention shall be described in detail with reference to the drawings. In the drawings, like reference numerals designate like structural elements.

It is to be noted that the embodiments are not limiting for the invention, but merely represent illustrative examples.

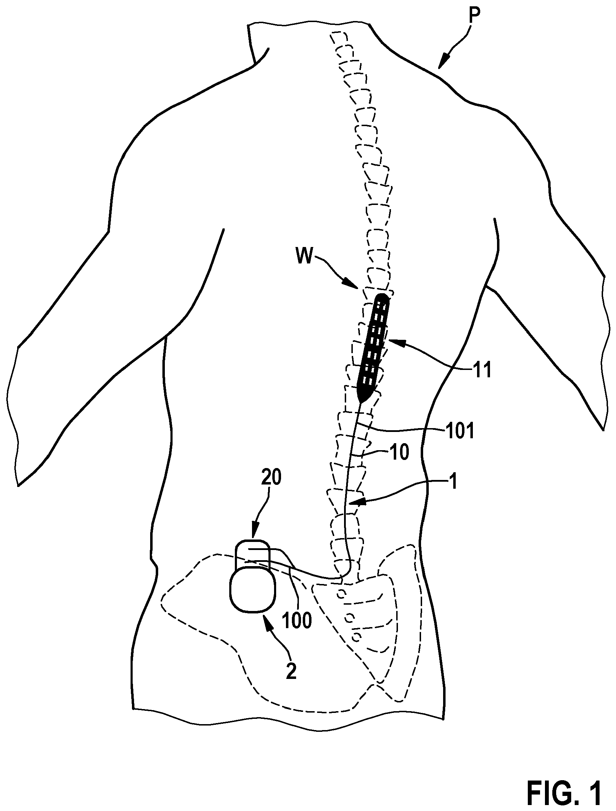

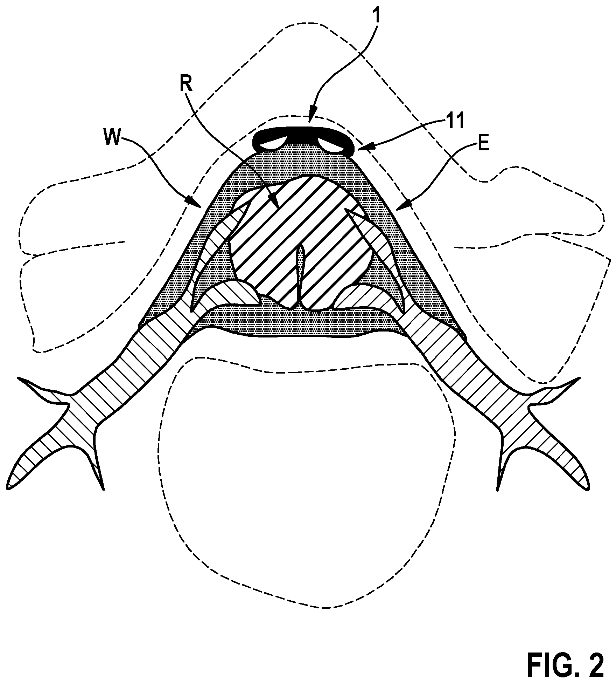

A medical electrode device 1, as shown in an embodiment in Figs. 1 and 2, is formed as a so-called paddle electrode and comprises a lead body 10 and a flattened electrode end 11 connected to the lead body 10 at a distal end 101 of the lead body 10, a plurality of contact elements being attached to the flattened electrode end 11 for injecting an electrical current e.g. in the region of the spinal column W of a patient P.

The electrode device 1 at a proximal end 100 of the lead body 10 is connected to a connector block 20 of a stimulation device 2, via which stimulation currents can be delivered to the electrode device 1 and output via the electrode arrangement arranged on the electrode end 11 to stimulate the spinal cord R in the region of the spinal column W.

As can be seen from the sectional view of Fig. 2, in the shown embodiment the electrode device 1 is implanted in the epidural space E in the region of the spinal column W of the patient P in such a way that the flattened electrode end 11 is located in the region of the spinal cord R and can thus introduce stimulation currents in a directed manner in order to effect nerve stimulation in the region of the spinal cord R.

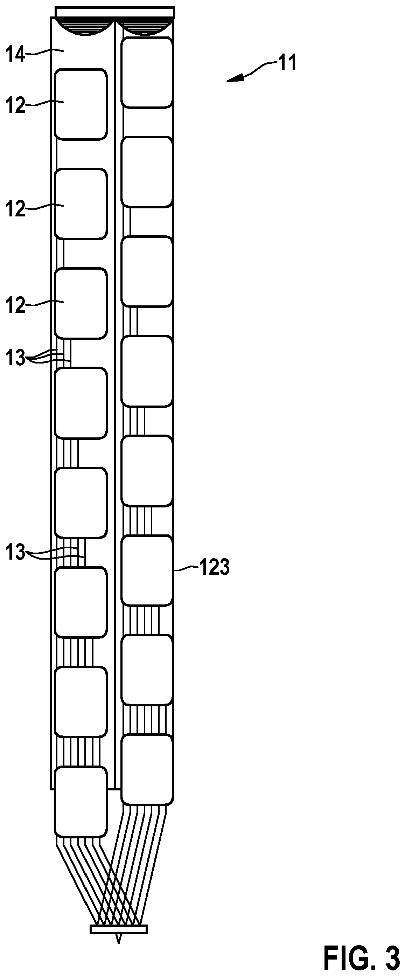

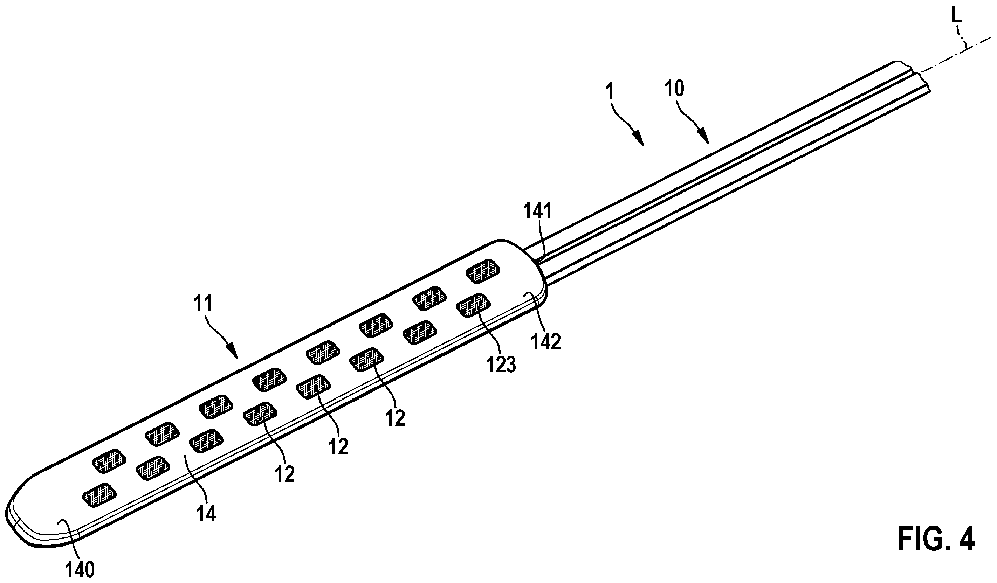

While the lead body 10 for example comprises a circular (isodiametric) cross-section, the electrode device 1 is flattened in the area of the flattened electrode end 11 which, as can be seen in Figs. 3 and 4, carries a plurality of contact elements 12 evenly or unevenly spaced on the flattened electrode end 11 in such a way that stimulation energy can be fed in a directed manner for example into the spinal cord R of a patient P.

As further illustrated in Fig. 3, each contact element 12 is connected to an electrical line 13, wherein each contact element 12 for example is connected to the stimulation device 2 via an associated, individual electrical line 13 and thus may be supplied with stimulation energy via the stimulation device 2 to emit electrical signals, or a group of electrical contact elements 12 is connected to a common electrical line 13 such that different groups of electrical contact elements 12 use different electrical lines 13. The electrical lines 13 are jointly routed as a single cable strand or, as illustrated in Fig. 4, as multiple cable strands in the lead body 10 in an encapsulated manner to the stimulation device 2.

As shown in Fig. 4, the contact elements 12 are arranged on a flat face 142 of a body 140 of a carrier element 14 and are exposed, in an implanted state, to tissue with electrical contact faces 123 facing outwards and can therefore come into electrical contact with surrounding tissue when the electrode device 1 is implanted in a patient. The flattened electrode end 11 at a proximal end 141 of the carrier element 14 is connected to the lead body 10.

In an electrode device 1 as shown in Figs. 1 to 4, an arrangement of contact elements 12 is arranged on a carrier element 14 of a flattened electrode end 11, the flattened electrode end 11 having a paddle-like shape for placement in the epidural space E in the vicinity of the spinal column W. The contact elements 12 herein are placed on and embedded in the carrier element 14, such that the contact elements 12 each face outwards and are exposed to tissue with their contact face 123.

For fabricating the electrode device 1, the electrical lines 13 are connected to the contact elements 12, wherein for example an individual electrical line 13 is connected to each contact element 12, as illustrated in Fig. 3. The contact elements 12, with the electrical lines 13 connected thereto, are placed on the carrier element 14 to form an arrangement of contact elements 12 for providing for a stimulation and/or sensing in an implanted state of the electrode device 1.

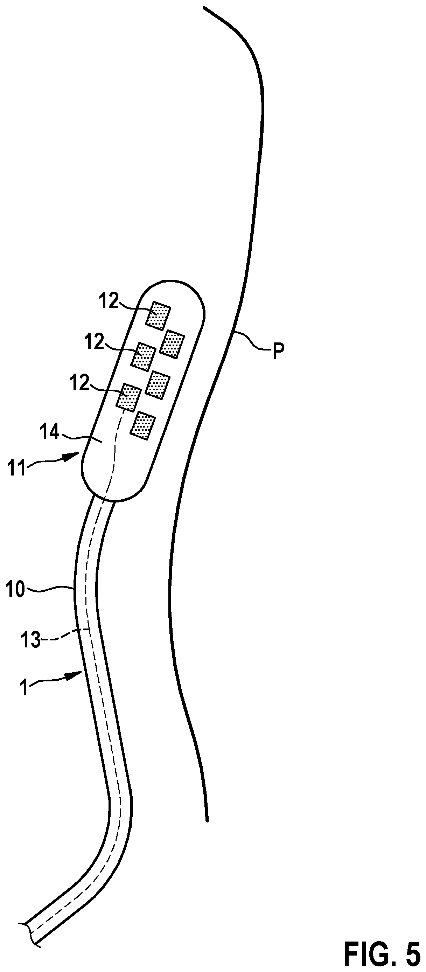

Referring now to Fig. 5, the one or the multiple electrical lines 13 extending along the lead body 10 together with the associated electrical contact elements 12 arranged on the carrier element 14 of the flattened electrode end 11 form an electrical structure having a substantial physical length. If the patient P carrying the medical electrode device 1 undergoes an MRI examination, an RF excitation field as produced by an MRI device may couple into the electrical structure as formed by the electrical lines 13 and the electrical contact elements 12, wherein the electrical structure may exhibit a resonant behavior causing a substantial field increase in the vicinity of the electrode device 1. As the electric contact elements 12 in the implanted position of the electrode device 1 are in contact with tissue in the surrounding of the flattened electrode end 11, this bears the risk of excessive heating in particular at the locations of the electrical contact elements 12.

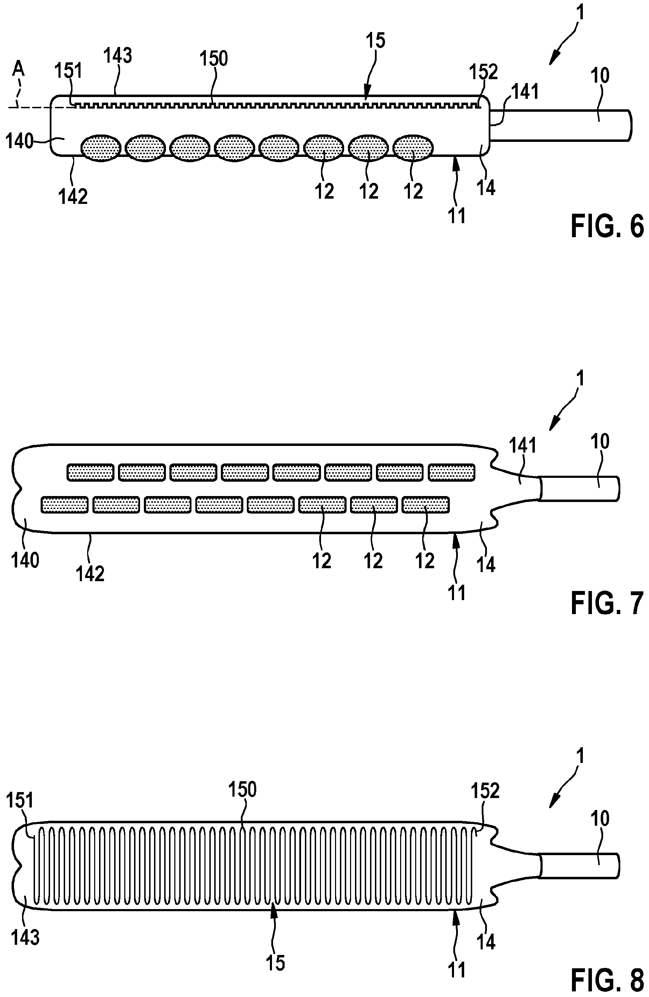

In order to prevent an excessive heating due to a coupling with RF excitation fields during an MRI examination, the flattened electrode end 11, in an embodiment as shown in Fig. 6, comprises an antenna element 15, which is embedded in the body 140 of the carrier element 14 and in a planar fashion extends along a flat plane A within the body 140.

The antenna element 15 is formed by an antenna line 150, which extends along a prescribed path (as it shall be explained in more detail below) and has a physical length which preferably corresponds to an electrical length exhibiting a series resonance at a defined frequency in the implanted state of the electrode device 1. In particular, the antenna element 15 may be designed such that the electrical length of the antenna line 150 corresponds to a half wavelength or an integer multiple of a half wavelength at the Lamor frequency at a predefined MRI magnetic field strength, for example 1.5 Tesla or 3 Tesla. This causes a strong coupling of the antenna element 15 to an RF excitation field during an MRI examination at the predefined magnetic field strength, such that RF energy predominantly couples into the antenna element 15, but less into the electrical structure formed by the electrical lines 13 and the electrical contact elements 12.

Hence, by using the antenna element 15 an energy coupling into the antenna element 15 may be achieved, causing an energy dissipation across the spatial extension of the structure of the antenna element 15. As the antenna element 15 is not in electrical contact with surrounding tissue and furthermore extends across the carrier element 14 of the flattened electrode end 11, energy is dissipated by the antenna element 15 across a substantial, large area, such that a local heating due to an excessive energy increase at a particular spot is prevented.

As visible from Fig. 6 in view of Fig. 7, the electrical contact elements 12 are arranged on a flat face 142 of the carrier element 14. The antenna element 15 is embedded within the body 140 of the carrier element 14, such that the antenna element 15 extends along a flat plane A in proximity to a face 143 of the carrier element 14 opposite to the flat face 142 at which the electrical contact elements 12 are arranged. By means of the antenna element 15, energy is hence dissipated towards the back of the flattened electrode end 11, that is towards the side of the flattened electrode end 11 opposite to the electrical contact elements 12.

Referring now to Figs. 8 to 13, the antenna line 150 forming the antenna element 15 may extend along a path exhibiting a multiplicity of turns such that the physical length of the antenna line 150 exceeds the dimensions of the antenna element 15 in the flat plane A.

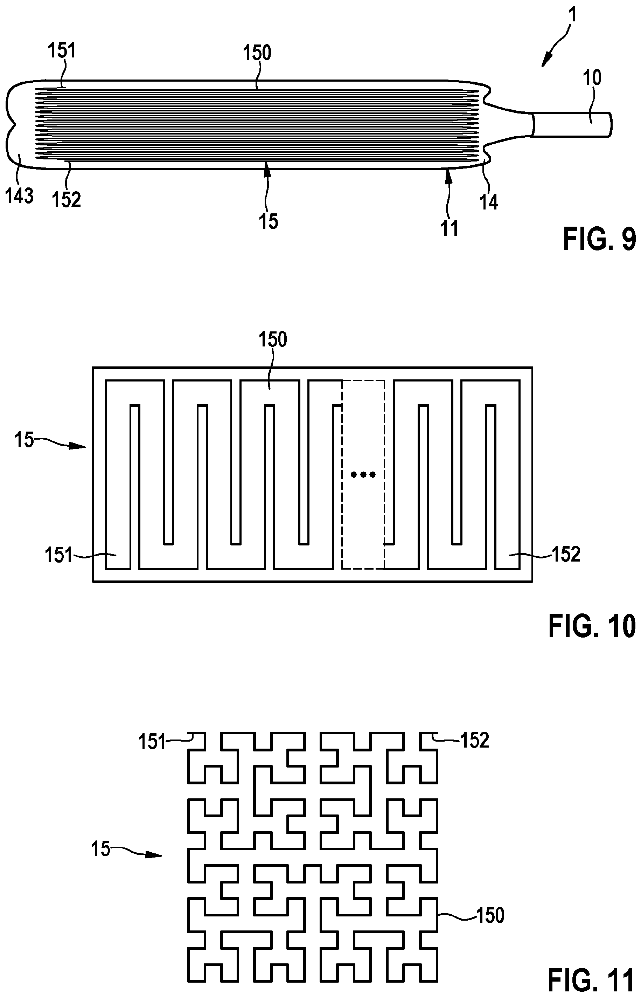

For example, the antenna line 150 may follow a meandering, curved path exhibiting a multiplicity of 180° turns (Fig. 8 and Fig. 9), wherein the antenna line 150 may meander across the width of the carrier element 14 (Fig. 8) or along the length of the carrier element 14 (Fig. 9). In the embodiments of Fig. 8 and Fig. 9, the antenna line 150 may for example be formed by a wire or a cable, which extends along the flat plane A corresponding to the plane of the drawing of Figs. 8 and 9.

In another embodiment, shown in Fig. 10, the antenna line 150 may for example be formed by a metallic foil which is cut by a laser to assume a meandering shape exhibiting a multiplicity of turns. The metallic foil may for example be a platinum foil, a tantalum foil, a titanium foil or a foil made from MP35N. The foil may have a thickness of 0.1 mm. Slits formed in the foil may have a width of 0.1 mm, and the conduction path formed by the foil may likewise have a width of for example 0.1 mm.

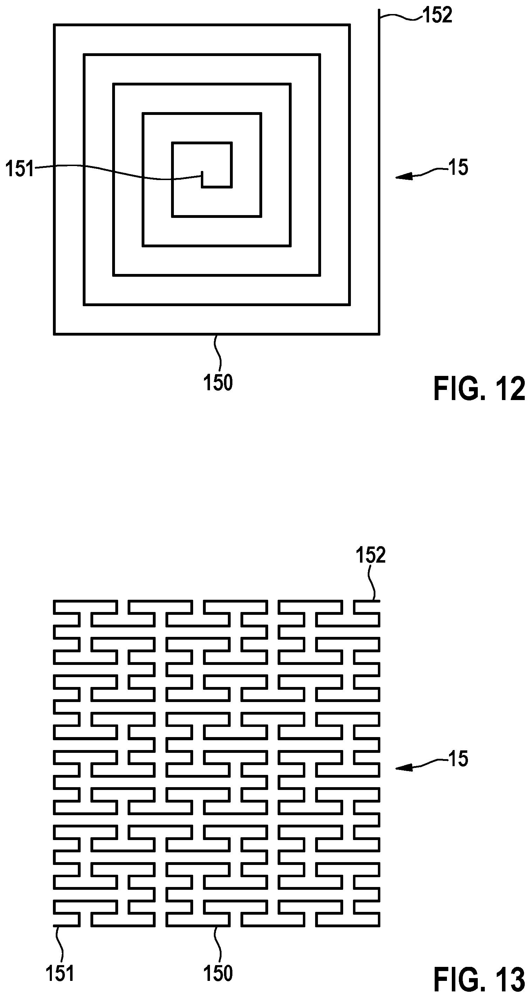

In yet another embodiment, the antenna line 150 may follow the path of a Hilbert curve (Fig. 11), a spiral path (Fig. 12) or the path of a Peano curve (Fig. 13). In each case the antenna line 150 flatly extends along the flat plane A (corresponding in each case to the plane of the drawing). The antenna line 150 may be formed e.g. by a wire or cable or by a conductive foil cut into shape for example by a laser.

A Hilbert curve (also known as Hilbert space-filling curve) is a continuous fractal space-filling curve. The Hilbert curve is constructed as a limit of piecewise linear curves. The length of the n-th curve is 2ⁿ-1/2ⁿ, i.e., the length grows exponentially with n even though each curve is contained in a square with area 1.

A Peano curve likewise is a space-filling curve. A Peano curve is a surjective, continuous function from the unit interval onto the unit square, however it is not injective. A Peano curve may be constructed by a sequence of steps, where the i-th step constructs a set of squares, and a sequence of the centers of the squares, from the set and sequence constructed in the previous step.

The antenna line 150 may be formed such that it exhibits an electrical series resonance for example at the Lamor frequency at a magnetic field strength of 1.5 Tesla or 3 Tesla. The electrical length of the antenna line 150 hence may correspond to a half wavelength or an integer multiple of a half wavelength at the defined Lamor frequency, e.g. 63.87 MHz (1.5 Tesla) or 127.74 MHz (3 Tesla). The electrical length herein is computed with respect to the implanted state of the electrode device 1, by assuming for example realistic conditions for the relative permittivity and the electrical conductivity in the implanted state. For example, for designing the length of the antenna line 150 a relative permittivity of 60 and an electrical conductivity of 0.47 S/m may be assumed.

The physical length of the antenna line 150 (as measured along its winding path) may in particular be larger than 150 mm, for example larger than 200 mm, for example larger than 300 mm.

The flattened electrode end 11 herein may have dimensions comprising for example a length (measured along a first direction pointing along the longitudinal axis L) in between 50 mm and 150 mm, for example 80 mm, a width (measured along a second direction transverse to the longitudinal axis L, wherein the first direction the second direction together span the flat plane A) in a range between 5 mm and 15 mm, for example 9 mm, and a thickness (measured along a third direction transverse to the first direction and to the second direction) smaller than the width.

The antenna element 15 may have dimensions corresponding to the dimensions of the flattened electrode end 11 along the first direction and the second direction but is designed such that it does not protrude from the flattened electrode end 11 and hence is embedded within the carrier element 14 and does not come into electrical contact with surrounding tissue in an implanted state of the electrode device 1.

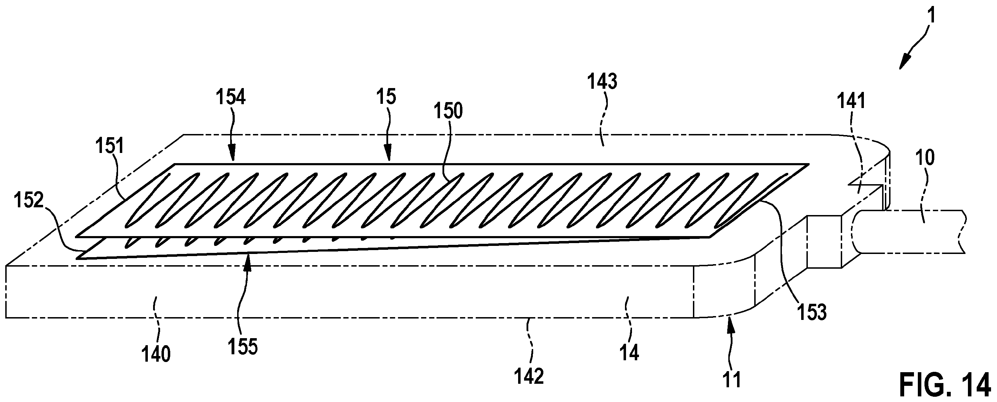

The antenna element 15 may, in one embodiment, comprise a single layer formed by the antenna line 150 extending along the flat plane A. In another embodiment, the antenna element 15 may comprise different layers 154, 155, as this is shown in an embodiment in Fig. 14. The layers 154, 155 are formed by the same, continuous antenna line 150, the layers 154, 155 being connected to one another at a fold line 153. The layers 154, 155 hence are folded with respect to one another, such that the layers 154, 155 extend in parallel to one another from the fold line 153.

In order to electrically insulate the layers 154, 155 of the antenna element 15 with respect to one another, the antenna line 150 may be formed by an electrically insulated, for example coated conduction path, for example a coated wire. In another embodiment, an electrically insulating foil, such as a plastics foil, may be placed in between the layers 154, 155, such that the layers 154, 155 are electrically connected to one another at the fold line 153, but not across their planar extension.

The antenna element 15 serves as a passive element within the flattened electrode end 11 to couple to an RF excitation field as used during an MRI examination. As the RF field predominantly couples into the antenna element 15, a field increase predominantly arises in the vicinity of the antenna element 15, wherein due to the large area that is spanned by the antenna element 15 an energy dissipation takes place across a substantial portion of the flattened electrode end 11. Hence, an excessive local heating is prevented. Furthermore, as the antenna element 15 is fully embedded within the carrier element 14 and is not in electrical contact with surrounding tissue, an excessive tissue heating due to a direct contact is prevented.

The antenna line 150 comprises two ends 151, 152 which are electrically open. The antenna element 15 serves as a passive element, which is not electrically connected to the electrical contact elements 12 or the electrical lines 13. Rather, the antenna element 15 is electrically (galvanically) separate from any other electrical structure of the electrode device 1.

The idea underlying the invention is not limited to the embodiments described above.

An electrode device as described herein may be used in a stimulation system, such as a neuro-stimulation system. However, it also is conceivable to use an electrode device as described herein for another stimulation device, such as a cardiac stimulation device.

The electrode device may comprise an arbitrary number of electrical contact elements, for example a number equal to or larger than four electrical contact elements. **In** one embodiment, the number of electrical contact elements placed on the flattened electrode end for contacting tissue is 16.

The electrode device may be designed for use in an MRI examination at 1.5 Tesla or 3 Tesla. As the lamor frequency at 3 Tesla is twice the lamor frequency at 1.5 Tesla, the electrode device may be usable for MRI examinations both at 1.5 Tesla and at 3 Tesla, without having to modify the design of the antenna element.

The electrode device may be designed also for other frequencies differing from the lamor frequency at 1.5 Tesla or 3 Tesla.

### List of Reference Numerals

- 1: Implantable electrode device
- 10: Lead body
- 100: Proximal end
- 101: Distal end
- 11: Electrode end
- 12: Contact element
- 123: Contact face
- 13: Electrical line
- 14: Carrier element
- 140: Body
- 141: Proximal end
- 142, 143: Face
- 15: Antenna element
- 150: Antenna line
- 151, 152: End of line
- 153: Fold line
- 154: First layer
- 155: Second layer
- 2: Stimulation device
- 20: Connector block
- A: Plane
- E: Epidural space
- L: Longitudinal axis
- P: Patient
- R: Spinal cord
- W: Spinal column

## Claims

1. A neuro-stimulation medical electrode device (1) for implantation into a patient (P), comprising:
a lead body (10) extending longitudinally along a longitudinal axis (L);
at least one electrical line (13) extending within the lead body (10); and
a flattened electrode end (11) arranged at a distal end of the lead body (10) and comprising a carrier element (14) and a multiplicity of electrical contact elements (12) connected to the at least one electrical line (13), wherein the multiplicity of electrical contact elements (12) is arranged on a flat face (142) of the carrier element (14) for contacting tissue in proximity to the flattened electrode end (11) in an implanted state of the medical electrode device (1),
wherein the flattened electrode end (11) further comprises an antenna element (15) arranged on the carrier element (14) and comprising an antenna line (150) having a first end (151) and a second end (152), wherein the antenna line (150) extends along a flat plane (A).

2. The neuro-stimulation medical electrode device (1) according to claim 1, wherein 2 said flat plane (A) extends substantially in parallel to said flat face (142) of the carrier element (14).

3. The neuro-stimulation medical electrode device (1) according to claim 1 or 2, wherein the antenna line (150) extends along a meandering path, a zig-zag path, a spiral path, a path of a Hilbert curve or a path of a Peano curve along said flat plane (A).

4. The neuro-stimulation medical electrode device (1) according to one of claims 1 to 3, wherein the antenna element (15) comprises a first layer (154) and a second layer (155) formed by said antenna line (150), wherein the first layer (154) and the second layer (155) at least partially extend in parallel to one another.

5. The neuro-stimulation medical electrode device (1) according to claim 4, wherein the first layer (154) and the second layer (155) are folded with respect to one another at a fold line (153), the first layer (154) and the second layer (155) extending in parallel to one another from the fold line (153).

6. The neuro-stimulation medical electrode device (1) according to one of the preceding claims, wherein the antenna line (150) is formed by a flexible conducting path, in particular made from a metallic foil.

7. The neuro-stimulation medical electrode device (1) according to one of the preceding claims, wherein the antenna line (150) comprises a physical length equal to or larger than 150 mm.

8. The neuro-stimulation medical electrode device (1) according to one of the preceding claims, wherein the electrical length of the antenna line (150), in a state corresponding to said implanted state of the medical electrode device (1), equals a half wavelength or an integer multiple of a half wavelength at the Lamor frequency at a predefined MRI magnetic field strength, in particular 1.5 Tesla or 3 Tesla.

9. The neuro-stimulation medical electrode device (1) according to one of the preceding claims, wherein the first end (151) and the second end (152) both are arranged on the carrier element (14) such that the antenna element (15) is confined to the flattened electrode end (11).

10. The neuro-stimulation medical electrode device (1) according to one of the preceding claims, wherein the antenna element (15) is electrically open at the first end (151) and the second end (152).

11. The neuro-stimulation medical electrode device (1) according to one of the preceding claims, wherein the antenna element (15) is electrically connected neither to the electrical contact elements (12) nor to the at least one electrical line (13).

12. The neuro-stimulation medical electrode device (1) according to one of the preceding claims, wherein the carrier element (14) comprises a body (140), wherein
the antenna element (15) is embedded in the body (14) such that the antenna element (15) is fully received within the body (140) and is electrically insulated towards the outside of the body (140).

13. The neuro-stimulation medical electrode device (1) according to claim 12, wherein the body (140) is made of an electrically insulating material, wherein the antenna element (15) is embedded in the material of the body (140).

14. The neuro-stimulation medical electrode device (1) according to one of the preceding claims, wherein said flat face (142) extends along a plane spanned by a first direction pointing along said longitudinal axis (L) and a second direction transverse to said longitudinal axis (L), wherein the carrier element (14) has a length, measured along the first direction, in a range between 50 mm and 150 mm, a width, measured along the second direction, between 5 mm and 15 mm and a thickness, measured along a third direction transverse to the first direction and the second direction, smaller than said width.

15. A method for fabricating a neuro-stimulation medical electrode device (1) for implantation into a patient (P), the method comprising:
providing a lead body (10) extending longitudinally along a longitudinal axis (L), at least one electrical line (13) extending within the lead body (10); and
arranging a flattened electrode end (11) at a distal end of the lead body (10), the flattened electrode end (11) comprising a carrier element (14) and a multiplicity of electrical contact elements (12) connected to the at least one electrical line (13), wherein the multiplicity of electrical contact elements (12) is arranged on a flat face (142) of the carrier element (14) for contacting tissue in proximity to the flattened electrode end (11) in an implanted state of the medical electrode device (1), wherein the flattened electrode end (11) further comprises an antenna element (15) arranged on the carrier element (14) and comprising an antenna line (150) having a first end (151) and a second end (152), wherein the antenna line (150) extends along a flat plane (A).

## Patentansprüche

1. Eine medizinische Neurostimulations-Elektrodenvorrichtung (1) zur Implantation in einen Patienten (P), umfassend:
einen Leitungskörper (10), der sich längs entlang einer Längsachse (L) erstreckt;
wenigstens eine elektrische Leitung (13), die sich innerhalb des Leitungskörpers (10) erstreckt; und
ein abgeflachtes Elektrodenende (11), das an einem distalen Ende des Leitungskörpers (10) angeordnet ist und ein Trägerelement (14) sowie eine Vielzahl von elektrischen Kontaktelementen (12) umfasst, die mit der wenigstens einen elektrischen Leitung (13) verbunden sind, wobei die Vielzahl von elektrischen Kontaktelementen (12) an einer flachen Fläche (142) des Trägerelements (14) angeordnet ist, um in einem implantierten Zustand der medizinischen Elektrodenvorrichtung (1) Gewebe in der Nähe des abgeflachten Elektrodenendes (11) zu kontaktieren,
wobei das abgeflachte Elektrodenende (11) ferner ein Antennenelement (15) umfasst, das an dem Trägerelement (14) angeordnet ist und eine Antennenleitung (150) mit einem ersten Ende (151) und einem zweiten Ende (152) umfasst, wobei sich die Antennenleitung (150) entlang einer flachen Ebene (A) erstreckt.

2. Die medizinische Neurostimulations-Elektrodenvorrichtung (1) nach Anspruch 1, **wobei** sich die flache Ebene (A) im Wesentlichen parallel zu der flachen Fläche (142) des Trägerelements (14) erstreckt.

3. Die medizinische Neurostimulations-Elektrodenvorrichtung (1) nach Anspruch 1 oder 2, **wobei** sich die Antennenleitung (150) entlang der flachen Ebene (A) entlang eines mäanderförmigen Pfads, eines Zickzackpfads, eines spiralförmigen Pfads, eines Pfads einer Hilbert-Kurve oder eines Pfads einer Peano-Kurve erstreckt.

4. Die medizinische Neurostimulations-Elektrodenvorrichtung (1) nach einem der Ansprüche 1 bis 3, **wobei** das Antennenelement (15) eine erste Lage (154) und eine zweite Lage (155) umfasst, die durch die Antennenleitung (150) gebildet sind, wobei sich die erste Lage (154) und die zweite Lage (155) zumindest teilweise parallel zueinander erstrecken.

5. Die medizinische Neurostimulations-Elektrodenvorrichtung (1) nach Anspruch 4, **wobei** die erste Lage (154) und die zweite Lage (155) gegenüber einander an einer Faltlinie (153) gefaltet sind, wobei sich die erste Lage (154) und die zweite Lage (155) von der Faltlinie (153) aus parallel zueinander erstrecken.

6. Die medizinische Neurostimulations-Elektrodenvorrichtung (1) nach einem der vorhergehenden Ansprüche, **wobei** die Antennenleitung (150) durch eine flexible Leiterbahn gebildet ist, insbesondere aus einer Metallfolie hergestellt ist.

7. Die medizinische Neurostimulations-Elektrodenvorrichtung (1) nach einem der vorhergehenden Ansprüche, **wobei** die Antennenleitung (150) eine physikalische Länge aufweist, die gleich oder größer als 150 mm ist.

8. Die medizinische Neurostimulations-Elektrodenvorrichtung (1) nach einem der vorhergehenden Ansprüche, **wobei** die elektrische Länge der Antennenleitung (150) in einem Zustand, der dem implantierten Zustand der medizinischen Elektrodenvorrichtung (1) entspricht, einer halben Wellenlänge oder einem ganzzahligen Vielfachen einer halben Wellenlänge bei der Lamor-Frequenz bei einer vorgegebenen MRT-Magnetfeldstärke, insbesondere 1,5 Tesla oder 3 Tesla, entspricht.

9. Die medizinische Neurostimulations-Elektrodenvorrichtung (1) nach einem der vorhergehenden Ansprüche, **wobei** das erste Ende (151) und das zweite Ende (152) beide derart an dem Trägerelement (14) angeordnet sind, dass das Antennenelement (15) auf das abgeflachte Elektrodenende (11) beschränkt ist.

10. Die medizinische Neurostimulations-Elektrodenvorrichtung (1) nach einem der vorhergehenden Ansprüche, **wobei** das Antennenelement (15) an dem ersten Ende (151) und dem zweiten Ende (152) elektrisch offen ist.

11. Die medizinische Neurostimulations-Elektrodenvorrichtung (1) nach einem der vorhergehenden Ansprüche, **wobei** das Antennenelement (15) weder mit den elektrischen Kontaktelementen (12) noch mit der wenigstens einen elektrischen Leitung (13) elektrisch verbunden ist.

12. Die medizinische Neurostimulations-Elektrodenvorrichtung (1) nach einem der vorhergehenden Ansprüche, **wobei** das Trägerelement (14) einen Körper (140) umfasst, wobei das Antennenelement (15) in den Körper (14) eingebettet ist, sodass das Antennenelement (15) vollständig innerhalb des Körpers (140) aufgenommen ist und gegenüber der Außenseite des Körpers (140) elektrisch isoliert ist.

13. Die medizinische Neurostimulations-Elektrodenvorrichtung (1) nach Anspruch 12, **wobei** der Körper (140) aus einem elektrisch isolierenden Material hergestellt ist, wobei das Antennenelement (15) in das Material des Körpers (140) eingebettet ist.

14. Die medizinische Neurostimulations-Elektrodenvorrichtung (1) nach einem der vorhergehenden Ansprüche, **wobei** sich die flache Fläche (142) entlang einer Ebene erstreckt, die durch eine erste Richtung, die entlang der Längsachse (L) weist, und eine zweite Richtung quer zu der Längsachse (L) aufgespannt ist, wobei das Trägerelement (14) eine Länge, gemessen entlang der ersten Richtung, in einem Bereich zwischen 50 mm und 150 mm, eine Breite, gemessen entlang der zweiten Richtung, zwischen 5 mm und 15 mm und eine Dicke, gemessen entlang einer dritten Richtung quer zu der ersten Richtung und der zweiten Richtung, kleiner als die Breite aufweist.

15. Ein Verfahren zur Herstellung einer medizinischen Neurostimulations-Elektrodenvorrichtung (1) zur Implantation in einen Patienten (P), wobei das Verfahren umfasst:
Bereitstellen eines Leitungskörpers (10), der sich längs entlang einer Längsachse (L) erstreckt, wobei sich wenigstens eine elektrische Leitung (13) innerhalb des Leitungskörpers (10) erstreckt; und
Anordnen eines abgeflachten Elektrodenendes (11) an einem distalen Ende des Leitungskörpers (10), wobei das abgeflachte Elektrodenende (11) ein Trägerelement (14) und eine Vielzahl von elektrischen Kontaktelementen (12) umfasst, die mit der wenigstens einen elektrischen Leitung (13) verbunden sind, wobei die Vielzahl von elektrischen Kontaktelementen (12) an einer flachen Fläche (142) des Trägerelements (14) angeordnet ist, um in einem implantierten Zustand der medizinischen Elektrodenvorrichtung (1) Gewebe in der Nähe des abgeflachten Elektrodenendes (11) zu kontaktieren, wobei das abgeflachte Elektrodenende (11) ferner ein Antennenelement (15) umfasst, das an dem Trägerelement (14) angeordnet ist und eine Antennenleitung (150) mit einem ersten Ende (151) und einem zweiten Ende (152) umfasst, wobei sich die Antennenleitung (150) entlang einer flachen Ebene (A) erstreckt.

## Revendications

1. Dispositif médical d'électrode de neurostimulation (1) destiné à être implanté dans un patient (P), comprenant :
un corps de sonde (10) s'étendant longitudinalement le long d'un axe longitudinal (L) ;
au moins une ligne électrique (13) s'étendant à l'intérieur du corps de sonde (10) ; et
une extrémité d'électrode aplatie (11) disposée à une extrémité distale du corps de sonde (10) et comprenant un élément support (14) et une pluralité d'éléments de contact électrique (12) reliés à l'au moins une ligne électrique (13), **dans lequel** la pluralité d'éléments de contact électrique (12) est disposée sur une face plane (142) de l'élément support (14) afin de venir en contact avec un tissu à proximité de l'extrémité d'électrode aplatie (11) dans un état implanté du dispositif médical d'électrode (1),
d**ans lequel** l'extrémité d'électrode aplatie (11) comprend en outre un élément d'antenne (15) disposé sur l'élément support (14) et comprenant une ligne d'antenne (150) présentant une première extrémité (151) et une seconde extrémité (152), **dans lequel** la ligne d'antenne (150) s'étend le long d'un plan (A).

2. Dispositif médical d'électrode de neurostimulation (1) selon la revendication 1, **dans lequel** ledit plan (A) s'étend sensiblement parallèlement à ladite face plane (142) de l'élément support (14).

3. Dispositif médical d'électrode de neurostimulation (1) selon la revendication 1 ou 2, **dans lequel** la ligne d'antenne (150) s'étend selon un trajet méandriforme, un trajet en zigzag, un trajet en spirale, un trajet suivant une courbe de Hilbert ou un trajet suivant une courbe de Peano le long dudit plan (A).

4. Dispositif médical d'électrode de neurostimulation (1) selon l'une des revendications 1 à 3, **dans lequel** l'élément d'antenne (15) comprend une première couche (154) et une seconde couche (155) formées par ladite ligne d'antenne (150), **dans lequel** la première couche (154) et la seconde couche (155) s'étendent au moins partiellement parallèlement l'une à l'autre.

5. Dispositif médical d'électrode de neurostimulation (1) selon la revendication 4, **dans lequel** la première couche (154) et la seconde couche (155) sont pliées l'une par rapport à l'autre au niveau d'une ligne de pliage (153), la première couche (154) et la seconde couche (155) s'étendant parallèlement l'une à l'autre à partir de la ligne de pliage (153).

6. Dispositif médical d'électrode de neurostimulation (1) selon l'une des revendications précédentes, **dans lequel** la ligne d'antenne (150) est formée par une piste conductrice flexible, notamment réalisée à partir d'une feuille métallique.

7. Dispositif médical d'électrode de neurostimulation (1) selon l'une des revendications précédentes, **dans lequel** la ligne d'antenne (150) présente une longueur physique supérieure ou égale à 150 mm.

8. Dispositif médical d'électrode de neurostimulation (1) selon l'une des revendications précédentes, **dans lequel** la longueur électrique de la ligne d'antenne (150), dans un état correspondant audit état implanté du dispositif médical d'électrode (1), est égale à une demi-longueur d'onde ou à un multiple entier d'une demi-longueur d'onde à la fréquence de Lamor pour une intensité prédéfinie du champ magnétique d'IRM, notamment 1,5 tesla ou 3 teslas.

9. Dispositif médical d'électrode de neurostimulation (1) selon l'une des revendications précédentes, **dans lequel** la première extrémité (151) et la seconde extrémité (152) sont toutes deux disposées sur l'élément support (14) de telle sorte que l'élément d'antenne (15) soit limité à l'extrémité d'électrode aplatie (11).

10. Dispositif médical d'électrode de neurostimulation (1) selon l'une des revendications précédentes, **dans lequel** l'élément d'antenne (15) est électriquement ouvert au niveau de la première extrémité (151) et de la seconde extrémité (152).

11. Dispositif médical d'électrode de neurostimulation (1) selon l'une des revendications précédentes, **dans lequel** l'élément d'antenne (15) n'est relié électriquement ni aux éléments de contact électrique (12) ni à l'au moins une ligne électrique (13).

12. Dispositif médical d'électrode de neurostimulation (1) selon l'une des revendications précédentes, **dans lequel** l'élément support (14) comprend un corps (140), **dans lequel** l'élément d'antenne (15) est noyé dans le corps (14) de telle sorte que l'élément d'antenne (15) soit entièrement reçu à l'intérieur du corps (140) et soit isolé électriquement vis-à-vis de l'extérieur du corps (140).

13. Dispositif médical d'électrode de neurostimulation (1) selon la revendication 12, **dans lequel** le corps (140) est constitué d'un matériau électriquement isolant, **dans lequel** l'élément d'antenne (15) est noyé dans le matériau du corps (140).

14. Dispositif médical d'électrode de neurostimulation (1) selon l'une des revendications précédentes, **dans lequel** ladite face plane (142) s'étend le long d'un plan défini par une première direction orientée le long dudit axe longitudinal (L) et une deuxième direction transversale audit axe longitudinal (L), **dans lequel** l'élément support (14) présente une longueur, mesurée selon la première direction, comprise entre 50 mm et 150 mm, une largeur, mesurée selon la deuxième direction, comprise entre 5 mm et 15 mm, et une épaisseur, mesurée selon une troisième direction transversale à la première direction et à la deuxième direction, inférieure à ladite largeur.

15. Procédé de fabrication d'un dispositif médical d'électrode de neurostimulation (1) destiné à être implanté dans un patient (P), le procédé comprenant :
la fourniture d'un corps de sonde (10) s'étendant longitudinalement le long d'un axe longitudinal (L), au moins une ligne électrique (13) s'étendant à l'intérieur du corps de sonde (10) ; et
la disposition d'une extrémité d'électrode aplatie (11) à une extrémité distale du corps de sonde (10), l'extrémité d'électrode aplatie (11) comprenant un élément support (14) et une pluralité d'éléments de contact électrique (12) reliés à l'au moins une ligne électrique (13), **dans lequel** la pluralité d'éléments de contact électrique (12) est disposée sur une face plane (142) de l'élément support (14) afin de venir en contact avec un tissu à proximité de l'extrémité d'électrode aplatie (11) dans un état implanté du dispositif médical d'électrode (1), **dans lequel** l'extrémité d'électrode aplatie (11) comprend en outre un élément d'antenne (15) disposé sur l'élément support (14) et comprenant une ligne d'antenne (150) présentant une première extrémité (151) et une seconde extrémité (152), **dans lequel** la ligne d'antenne (150) s'étend le long d'un plan (A).
